# EUROPÄISCHE PATENTSCHRIFT

(11) **EP 1 042 066 B1**
(45) Veröffentlichungstag und Bekanntmachung des Hinweises auf die Patenterteilung: **06.03.2002**
(21) Anmeldenummer: 98965863.8
(22) Anmeldetag: 21.12.1998
(51) Int. Cl.: B01J 23/72, B01J 37/02, B01J 37/08, C07C 45/00

(54) **VERFAHREN ZUR HERSTELLUNG OXIDISCHER KATALYSATOREN, DIE KUPFER IN EINER OXIDATIONSSTUFE GRÖSSER ALS 0 ENTHALTEN**
METHOD FOR PRODUCING OXIDIC CATALYSTS CONTAINING COPPER WITH AN OXIDATION NUMBER GREATER THAN 0
PROCEDE DE PREPARATION DE CATALYSEURS DE TYPE OXYDIQUE CONTENANT DU CUIVRE AVEC UN NOMBRE D'OXYDATION SUPERIEUR A 0

(30) Priorität: 22.12.1997 DE 19757297
(43) Veröffentlichungstag der Anmeldung: 11.10.2000
(73) Patentinhaber: BASF AKTIENGESELLSCHAFT, 67056 Ludwigshafen (DE)
(72) Erfinder: HEINEKE, Daniel, D-67487 Maikammer (DE); MEISSNER, Ruprecht, D-67273 Weisenheim (DE); HESSE, Michael, D-67549 Worms (DE); GEHRKEN, Henning-Peter, D-48147 Münster (DE)
(74) Vertreter: Kinzebach, Werner, Dr.
(86) Internationale Anmeldenummer: EP9808388
(87) Internationale Veröffentlichungsnummer: WO9932224

(56) Entgegenhaltungen:
- EP-A- 0 569 077
- EP-A- 0 653 243
- WO-A-98/10864
- JEON G S ET AL: "ACTIVE AND SELECTIVE COPPER CATALYSTS SUPPORTED ON ALKALI-DOPED SILICA FOR THE DEHYDROGENATION OF CYCLOHEXANOL TO CYCLOHEXANONE" KOREAN JOURNAL OF CHEMICAL ENGINEERING, Bd. 12, Nr. 1, 1995, Seite 132/133 XP000613504
- JEON G S ET AL: "PREPARATION AND CHARACTERIZATION OF SILICA-SUPPORTED COPPER CATALYSTS FOR THE DEHYDROGENATION OF CYCLOHEXANOL TO CYCLOHEXANONE" APPLIED CATALYSIS A: GENERAL, Bd. 115, Nr. 1, August 1994, Seiten 29-44, XP000613390 in der Anmeldung erwähnt
- DATABASE WPI Section Ch, Week 7606 Derwent Publications Ltd., London, GB; Class E15, AN 76-10700X XP002099714 & SU 465 217 A (AS BELO PHYS ORG CH) , 14. August 1975 in der Anmeldung erwähnt

## Beschreibung

Die vorliegende Erfindung betrifft ein Verfahren zur Herstellung von oxidischen Katalysatoren, die Kupfer in einer Oxidationsstufe > 0 enthalten, durch Behandeln eines festen, oxidischen Trägermaterials mit einer wässrigen Kupfersalzlösung und anschließendes Kalzinieren. Die vorliegende Erfindung betrifft auch die nach diesem Verfahren erhältlichen Katalysatoren sowie deren Verwendung zur Dehydrierung von sekundären Alkoholen zu den Ketonen, insbesondere zur Dehydrierung von Cyclohexanol zu Cyclohexanon.

Die katalytische Dehydrierung von sekundären Alkoholen spielt bei der großtechnischen Herstellung von Ketonen, beispielsweise bei der Herstellung von Cyclohexanon aus Cyclohexanol, eine wichtige Rolle (siehe z. B. K. Weissermel, H.-J. Arpe, Industrielle Organische Chemie, 4. Aufl. Verlag Chemie, Weinheim, 1994, S. 274). Diese Verfahren sind, sieht man von der erfindungsgemäßen Ausführungsform ab, in zahlreichen Varianten bekannt und es bedarf daher lediglich der prinzipiellen Erläuterung. Üblicherweise wird in den Verfahren das Alkanol bzw. ein Alkanol/Keton-Gemisch an einen kupferhaltigen Kontakt bei erhöhter Temperatur, in der Regel oberhalb 200 °C, geführt. Hierbei ist prinzipiell zu berücksichtigen, dass die katalytische Dehydrierung von Alkoholen zu Ketonen reversibel ist und die Lage des Gleichgewichts sich mit abnehmender Temperatur zu Gunsten der Edukte verschiebt. Ferner ist die Gleichgewichtseinstellung bei niedriger Temperatur verlangsamt, so dass in der Regel nur niedrige Umsätze erzielt werden können. Führt man hingegen das Verfahren bei erhöhter Temperatur, z. B. oberhalb 400 °C, durch, werden geringere Wertproduktselektivitäten erreicht, da bei diesen Temperaturen zum Teil erhebliche Nebenreaktionen, z. B. Dehydratisierung der Alkohole oder Dimerisierung der gebildeten Ketone, stattfinden.

Für die Dehydrierung von Alkanolen bei Temperaturen unterhalb 400 °C werden häufig Katalysatoren eingesetzt, die Kupfer als Aktivkomponente auf einem festen, meist oxidischen Träger enthalten. Der Kupfergehalt derartiger Katalysatoren kann bis zu 50 Gew.-%, bezogen auf das Gesamtgewicht des Katalysators, betragen. Typische Trägermaterialien oxidischer Natur sind keramische Oxide wie Siliziumdioxid, z.B. Kieselsäure, Silikate, Alumosilikate, Aluminiumoxid, Zirkondioxid und Titandioxid, ferner Zeolite und Bimsstein. Neben Kupfer als Aktivkomponente enthalten die Katalysatoren des Standes der Technik häufig in geringer Menge Alkalimetalle als Promotoren.

Aus der GB-A- 1081491 ist Cu/Al₂O₃, aus der SU-A 465 217 Cu/Li/ SiO₂ und aus der SU-A 522853 Cu/K/Al₂O₃ zur nichtoxidativen Dehydrierung von Cyclohexanol bekannt. Die betreffenden Kupferkatalysatoren werden meist so hergestellt, dass die Kupfer-Aktivkomponente entweder auf einen vorgefertigten Träger durch Fällung eines Kupfer-Salzes oder durch Tränkung mit einer geeigneten Kupfer-Salz-Lösung aufgebracht wird oder die Komponenten, aus denen der Katalysator zusammengesetzt ist, gemeinsam gefällt werden.

Chang et al. (Appl. Catal. A 103 (1994), S. 233-42) beschreiben Kupfer-Katalysatoren für die Dehydrierung von Cyclohexanol zu Cyclohexanon, die durch reduktive Fällung des Kupfers auf α-Al₂O₃ als Träger erhältlich sind. Die reduktive Fällung hat den Nachteil, dass in der Regel der Träger zunächst mit einem Edelmetall wie Platin, Rhodium, Iridium, Gold oder Palladium geimpft werden muss, um eine gleichmäßige Abscheidung des Kupfers auf dem Träger zu erreichen. Hierdurch entstehen zusätzliche Kosten. Zudem handelt es sich bei dem von Chang et al. beschriebenen Katalysatoren um schlecht tablettierbare Pulver, die nur bedingt eine Verarbeitung zu Formkörpern wie Tabletten erlauben. Für eine großtechnische Anwendung kommen daher diese Katalysatoren nicht in Frage.

Die EP-A-0 569 077 beschreibt ein Verfahren zur Herstellung von Metall-Trägerkatalysatoren, bei dem man in einer Lösung einer Metallverbindung einen Träger, der mit der Metallverbindung reagieren kann, suspendiert und außerdem ein wasserlösliches Polymer zusetzt, das ebenfalls mit dem Träger reagieren kann. Der so erhaltene Feststoff wird anschließend thermisch behandelt. Als Metallverbindungen werden u. a. Kupfersalze genannt.

Die EP-A-0 653 243 betrifft ein Verfahren zur Herstellung von Trägerkatalysatoren. Man erhält die betreffenden Katalysatoren, indem man eine Lösung der katalytisch aktiven Metall-Komponente oder ihrer Vorläuferverbindung mit einer in Wasser quellbaren vernetzten polymeren Verbindung versetzt. Anschließend wird das gequollene Polymer mit einem pulverförmigen Trägermaterial vermischt, zu Formkörpern verarbeitet, getrocknet und calciniert.

Ferner werden von Chung et al. (Appl. Catal. A 115 (1994), S. 29-44) Kupfer-Katalysatoren beschrieben, die durch Alkali-Fällung von Kupfer aus einer wässrigen Kupfer-Salz-Lösung auf einen Siliziumdioxid-Träger erhältlich sind. Die nach dem dort beschriebenen Verfahren erhältlichen Katalysatoren zeichnen sich zwar durch eine vergleichsweise hohe Selektivität aus, ihre Aktivität ist jedoch für die gewünschten Anwendungen zu gering.

Darüberhinaus verringert sich bei den Katalysatoren des Standes der Technik mit zunehmender Standzeit die Aktivität. Dies hat zur Folge, dass bei längerer Betriebsdauer die Betriebstemperatur des Reaktors ständig angehoben werden muss und damit ein Selektivitätsverlust eintritt. Zudem beschleunigt die Temperaturanhebung die Desaktivierung des Katalysators.

Der vorliegenden Erfindung liegt somit die Aufgabe zu Grunde, einen Katalysator für die hichtoxidative Dehydrierung von sekundären Alkoholen zu den entsprechenden Ketonen bereitzustellen, der eine hohe Aktivität bei gleichzeitig hoher Selektivität aufweist. Zudem sollte der Katalysator bei längerer Betriebsdauer seine Aktivität nicht verlieren. Ferner sollte der Katalysator kostengünstig erhältlich sein und vorteilhafte mechanische Eigenschaften aufweisen.

Diese Aufgabe konnte durch Katalysatoren gelöst werden, die durch Behandeln eines festen, oxidischen Trägermaterials mit wässrigen Kupfer-Salz-Lösungen, die wenigstens ein organisches, wasserlösliches Polymer, welches Kupferionen koordinativ bindet, enthält, und anschließendes Kalzinieren erhältlich sind.

Demnach betrifft die vorliegende Erfindung ein Verfahren zur Herstellung von oxidischen Katalysatoren, die Kupfer in einer Oxidationsstufe > 0 enthalten, durch Behandeln eines festen, oxidischen Trägermaterials mit einer wässrigen Lösung, die wenigstens ein Kupfersalz enthält, und anschließendes Kalzinieren, dadurch gekennzeichnet, dass das Kupfer durch Fällung aus der wässrigen Lösung mittels eines Fällungsmittels auf das feste, oxidische Trägermaterial aufgebracht wird, wobei die wässrige Lösung wenigstens ein organisches, wasserlösliches Polymer in einer Konzentration von 0,1 bis 100 g/l enthält, welches Kupferionen koordinativ bindet und das ausgewählt ist unter Homo- und Copolymeren des N-Vinylpyrrolidons. Die vorliegende Erfindung betrifft auch die durch dieses Verfahren erhältlichen Katalysatoren.

Die wasserlöslichen Polymere enthalten in der Regel wenigstens 50 Mol-% N-Vinyllactame wie N-Vinyl-pyrrolidon. Geeignete Comonomere für N-Vinylpyrrolidon sind ethylenisch ungesättigte Carbonsäuren wie Acrylsäure, Methacrylsäure, Crotonsäure, Maleinsäure, Fumarsäure und Itaconsäure, deren Amide, deren Hydroxyalkylester, Vinylacetat, Vinylpropionat und Stickstoff enthaltende, vinylsubstituierte Heterocyclen wie Vinylpyridine und Vinylimidazol.

Die erfindungsgemäß zur Anwendung kommenden wasserlöslichen Polymere weisen in der Regel ein gewichtsmittleres Molekulargewicht M_{w} > 500 Dalton und bis 10⁶ Dalton auf.

Bevorzugt sind die Bomo- und Copolymere, insbesondere die Homopolymere, mit einem gewichtsmittleren Molekulargewicht im Bereich von 1000 bis 100 000, insbesondere im Bereich von 2000 bis 50 000 und ganz besonders bevorzugt im Bereich von 5000 bis 30 000. Homo- und Copolymere von N-Vinyllactamen sind dem Fachmann hinreichend bekannt, beispielsweise aus Ullmann's Encyclopedia of Industrial Chemistry, 5th ed, Vol A21, S. 752-756 sowie aus Römpp, Chemielexikon, 9. Aufl., Georg-Thieme-Verlag, Stuttgart, 1989-1992, S. 3583f. sowie dort zit. Lit. (siehe auch Davidson, Handbook of Water-Soluble Gums and Resins, McGraw-Hill, New York-London 1980, S.21.1-21.21; Houben-Weyl, E20/2, 1267-1276).

Im erfindungsgemäßen Verfahren wird das Kupfer auf den festen, oxidischen Träger durch Behandeln des Trägers mit einer wässrigen Lösung, die wenigstens ein Kupfersalz und wenigstens ein wasserlösliches Polymer in der oben angegebenen Menge enthält, aufgebracht. Das Aufbringen erfolgt in einer weise, dass sich das Kupfer auf dem festen oxidischen Träger abscheidet, wobei die Oxidationsstufe des abgeschiedenen Kupfers > 0 ist.

Im erfindungsgemäßen Verfahren wird das Kupfer durch Ausfällen des Kupfers auf den festen, oxidischen Träger mittels eines Fällungsmittels aufgebracht. Hierbei geht man in der Regel so vor, dass man den festen oxidischen Träger in einer wässrigen Lösung, die wenigstens ein Kupfer-Salz und wenigstens ein wasserlösliches Polymer enthält, suspendiert und anschließend ein Fällungsmittel zusetzt. Selbstverständlich können das Kupfer-Salz als auch das wasserlösliche Polymer in gelöster Form oder als Feststoffe zu einer wässrigen Suspension des festen, oxidischen Trägers gegeben werden. Erfindungswesentlich ist, daß das Polymer und das Kupfer-Salz vor der Zugabe des Fällungsmittels in der wässrigen Dispersion des Trägers in gelöster Form zugegen sind. Im Anschluss an die Fällung wird der hierbei erhaltene Feststoff (Trägermaterial mit ausgefällter Kupferverbindung) abfiltriert und getrocknet. Gegebenenfalls wird der so erhaltene Feststoff vor der Trocknung mit Wässer oder mit einem mit Wasser mischbaren organischen Lösungsmittel zur Entfernung von überschüssigem Fällungsmittel gewaschen. Die Trocknung erfolgt in der Regel bei Temperaturen oberhalb 100 °C bei Normaldruck. Sie kann auch bei reduziertem Druck und gegebenenfalls niedriger Temperatur erfolgen. Die Trocknungsdauer beträgt in der Regel 1 bis 48 h. Die Trocknungstemperatur wird in der Regel 200 °C nicht überschreiten.

Geeignete Fällungsmittel sind wässrige Lösungen von wasserlöslichen Salzen, die Phosphat-, Sulfid-, Carbonat-, Oxalat- oder Hydroxid-Ionen in einer für die Fällung hinreichenden Konzentration enthalten. Durch Zugabe des Fällungsmittels wird das Kupfer als schwerlösliches Salz auf dem festen, oxidischen Träger abgeschieden. Im Unterschied zur stromlosen Verkupferung liegt hier das Kupfer in einer Oxidationsstufe > 0 vor. Typische Fällungsmittel sind die Alkalimetallsalze der vorgenannten Anionen, insbesondere die Natrium- und Kalium-Salze. Sulfidionen können auch in Form von Schwefelwasserstoff eingesetzt werden.

Bevorzugte Fällungsmittel sind wässrige Lösungen von wasserlöslichen Carbonaten und von Hydroxiden, beispielsweise wässrige Lösungen von Natriumcarbonat, Kaliumcarbonat, Natriumhydroxid und Kaliumhydroxid. Ganz besonders bevorzugt werden als Fällungsmittel wässrige Lösungen des Natriumcarbonats oder des Kaliumcarbonats eingesetzt.

Die Fällung kann sowohl bei Raumtemperatur als auch bei erhöhter Temperatur durchgeführt werden. Vorzugsweise führt man die Fällung bei Temperaturen oberhalb 50 °C durch, insbesondere wenn man als Fällungsmittel Carbonat oder Hydroxid einsetzt. Vorzugsweise wird man das Fällungsmittel nicht auf einmal, sondern über einen längeren Zeitraum, vorzugsweise innerhalb 0,5 bis 10 h, insbesondere innerhalb 1 bis 5 h zugeben.

Für das erfindungsgemäße Verfahren kommen in der Regel oxidische Trägermaterialien zur Anwendung, die ausgewählt sind unter keramischen Oxiden wie Siliziumdioxid, z.B. Kieselsäuren und Kieselgelen, Silikaten, Alumosilikaten, Aluminiumoxid, insbesondere α-Aluminiumoxid, Zirkondioxid und Titandioxid oder Mischungen der vorgenannten Oxide, ferner Zeolithen und Bimsstein. Erfindungsgemäß bevorzugte Trägermaterialien enthalten wenigstens 80 Gew.-% Siliziumdioxid, beispielsweise in Form eines Kieselgels oder einer Kieselsäure. Die erfindungsgemäß zur Anwendung kommenden oxidischen Trägermaterialien weisen in der Regel eine spezifische BET-Oberfläche (gemessen nach DIN 66131) oberhalb 30 m²/g, vorzugsweise oberhalb 50 m²/g und insbesondere oberhalb 100 m²/g auf. In der Regel wird die BET-Oberfläche 600 m²/g und insbesondere 500 m²/g nicht überschreiten. In einer speziellen Ausführungsform der vorliegenden Erfindung verwendet man ein Siliziumdioxid als oxidisches Trägermaterial, das eine BET-Oberfläche im Bereich von 200 bis 400 m²/g aufweist.

Die für das erfindungsgemäße Verfahren eingesetzten wässrigen Kupfer-Salz-Lösungen enthalten in der Regel ein wasserlösliches Kupfer-Salz in einer Konzentration von 0,05 bis 5 Mol/l, vorzugsweise 0,1 bis 3 Mol/l (bezogen auf die wässrige Phase vor Zugabe des Fällungsmittels bzw. auf die für die Imprägnierung verwendete Kupferlösung). Hierbei richtet sich die Konzentration in der Regel nach der Löslichkeit des Kupfer-Salzes oder orientiert sich an Praktikabilitätsgründen. Typische Kupfer-Salze sind Kupfer(II)acetat, Kupfer(II)chlorid, Kupfer(II)sulfat, Kupfer(II)nitrat sowie die entsprechenden Hydrate dieser Salze. Die Konzentration des wasserlöslichen Polymeren in diesen Lösungen liegt in der Regel im Bereich von 0,1 bis 100 g/l, vorzugsweise im Bereich von 0,5 bis 50 g/l und besonders bevorzugt im Bereich von 1 bis 10 g/l. Das Verhältnis von Kupfer zu Polymer liegt in der Regel im Bereich von 100:1 bis 1:2, vorzugsweise im Bereich von 50:1 bis 1:1 und insbesondere 20:1 bis 2:1 (Gewichtsverhältnis von Kupfer:Polymer).

An die erfindungsgemäße Behandlung des festen, oxidischen Trägermaterials mit der wässrigen Kupfer-Salz-Lösung schließt sich gegebenenfalls ein Kalzinierungsschritt an. Die Kalzinierung erfolgt vorzugsweise bei Temperaturen im Bereich von 250 bis 450 °C an Luft oder in einer Inertgasatmosphäre, zweckmäßig in Stickstoff. Die Kalzinierungsdauer beträgt in der Regel 1 bis 24 h.

Das aus dem erfindungsgemäßen Verfahren erhaltene Katalysatorpulver wird vorzugsweise, im Allgemeinen unter Zumischung von Tablettierhilfsstoffen, zu Formkörpern wie Tabletten, Strängen, Ringen, Wagenrädern, Sternen, Monolithen, Kugeln, Splitt oder Extrudaten, bevorzugt zu Tabletten, verpresst. Als Tablettierhilfsstoffe kann man die üblicherweise verwendeten Tablettierhilfsstoffe verwenden. Beispielhaft seien Graphit, Magnesiumstearat, Methylcellulosen (wie Walocel®), Cu-Pulver oder Mischungen davon genannt. Die Herstellung von Formkörpern kann vor oder nach dem Kalziniervorgang erfolgen.

Den Cu-Gehalt des Katalysators (berechnet als metallisches Kupfer) wählt man üblicherweise im Bereich von 0,01 bis 50 Gew.-%, bevorzugt von 2 bis 30 Gew.-%, besonders bevorzugt von 5 bis 20 Gew.-%, bezogen auf das Gesamtgewicht der Katalysatormasse. Herstellungsbedingt können die erfindungsgemäßen Katalysatoren bis zu 2 Gew.-% Alakalimetalle, insbesondere Natrium oder Kalium enthalten. Die BET-Oberfläche (gemessen nach DIN 66131) des Katalysators liegt in der Regel nicht unter 30 m²/g, bevorzugt im Bereich von 50 bis 600 m²/g, besonders bevorzugt im Bereich von 100 bis 500 m²/g. In einer speziellen Ausführungsform beträgt sie 200 bis 400 m²/g. Die mittlere Partikelgröße des auf dem Katalysatoträger erfindungsgemäß abgeschiedenen Kupfersalzes liegt in der Regel unterhalb 20 nm. Die Partikelgröße kann beispielsweise durch Transmissionselektronenmikroskopie oder durch Bestimmung der Größe der kristallinen Bereiche mittels Röntgenbeugungsanalyse (XRD) erfolgen.

Die erfindungsgemäßen Katalysatoren können für die Dehydrierung sekundärer Alkohole zu den entsprechenden Ketonen eingesetzt werden. Bei den sekundären Alkoholen kann es sich sowohl um offenkettige Alkohole handeln als auch um Cycloalkanole. Bevorzugte Edukte für das erfindungsgemäße Verfahren sind Alkanole und Cycloalkanole mit 3 bis 14 Kohlenstoffatomen. Besonders bevorzugte Edukte sind Cycloalkanole wie Cyclopentanol, Cyclohexanol, Methylcyclohexanole und Cyclododecanol. Ganz besonders geeignet sind die erfindungsgemäßen Katalysatoren für die Dehydrierung von Cyclohexanol.

Als Edukte für die Dehydrierung können sowohl die reinen Alkohole als auch Mischungen von verschiedenen Alkoholen eingesetzt werden. Häufig werden auch Mischungen aus Alkohol und dem Dehydrierungsprodukt eingesetzt. Als Edukt für die Dehydrierung von Cyclohexanol wird im Allgemeinen ein Gemisch aus Cyclohexanol und Cyclohexanon eingesetzt. Selbstverständlich kann man auch reines Cyclohexanol einsetzen. Üblicherweise besteht das einzusetzende Gemisch aus 50 bis 100, vorzugsweise 60 bis 99, insbesondere 96 Gew.-% Cyclohexanol und 50 bis 0, vorzugsweise 40 bis 1, insbesondere 4 Gew.-% Cyclohexanon. Cyclohexanon und Cyclohexanol erhält man üblicherweise durch Oxidation von Cyclohexan und anschließender Aufkonzentrierung des Cyclohexanols durch destillative Entfernung von Cyclohexanon und anderen leichtsiedenden Komponenten.

Die Dehydrierung der sekundären Alkohole zu den Ketonen wird im Allgemeinen in der Gasphase bei Temperaturen von 180 bis 400 °C, bevorzugt im Bereich von 200 bis 350 °C, besonders bevorzugt im Bereich von 220 bis 260 °C durchgeführt. Den Druck wählt man dabei in der Regel im Bereich von 50 kPa bis 5 MPa, insbesondere arbeitet man unter Atmosphärendruck.

In der Regel aktiviert man den Katalysator vor der eigentlichen Reaktion mit Wasserstoff (Formierungsphase). Dabei wird im Allgemeinen so vorgegangen, dass ein mit Inertgas, bevorzugt Stickstoff, verdünnter Wasserstoffstrom bei einer bestimmten Temperatur, vorzugsweise im Bereich von 120 bis 300 °C, über den Katalysator gefahren wird. Der Wasserstoffanteil im Reduktionsgas wird dann üblicherweise kontinuierlich erhöht, bis keine signifikante Temperaturveränderung mehr eintritt.

In einer bevorzugten Ausführungsform fährt man das Edukt gasförmig über den Katalysator, wobei die LHSV (Liquid Hourly Space Velocity) vorzugsweise von 0,1 bis 100 h⁻¹, besonders bevorzugt von 0,1 bis 20 h⁻¹ beträgt. Das Edukt kann mit einem Inertgas wie Stickstoff oder mit Dampf vermischt werden. Das Produkt der Dehydrierung kann wie üblich (für Cyclohexanon siehe beispielsweise DE-A 1,296,625 und DE-A 1,443,462) aufgearbeitet und weiterverarbeitet werden.

In einer weiteren bevorzugten Ausführungsform trennt man aus dem die Reaktionszone verlassenden Reaktionsgemisch den Wasserstoff ab und setzt ihn der die Reaktionszone eintretenden Gasmischung zu. Des weiteren ist es vorteilhaft, das Reaktionsgemisch so lange im Kreis zu fahren,'bis der gewünschte Umsatz erreicht ist.

Der erfindungsgemäße Katalysator kann aufgrund seiner hohen Aktivität bei wesentlich tieferen Temperaturen betrieben werden als großtechnisch eingesetzte Katalysatoren, weist kurze Formierungsphasen, hohe Selektivität und Umsätze nahe des Gleichgewichtes auf. Darüberhinaus tritt eine merkliche Desaktivierung erst nach deutlich längeren als bisher üblichen Zeiträumen bei bekannten Katalysatoren auf.

Der erfindungsgemäße Katalysator zeichnet sich durch eine gute Tablettierbarkeit, ausreichende Härte, hohe Umsätze bei niedrigen Betriebstemperaturen, hohe Selektivitäten zu Cyclohexanol und eine gute Standzeit aus.

### Beispiele

### I. Herstellung der erfindungsgemäßen Katalysatoren (Beispiel 1)

271 g Siliciumdioxid mit einer BET-Oberfläche von 270 m²/g wurden in ein Liter entionisiertem Wasser suspendiert. Hierzu gab man 5 g Polyvinylpyrrolidon (PVP der Fa. Merck, Bestellnr. 7443, mittleres Molekulargewicht 25 000 g/mol) und 339 ml einer 2M, wässrigen Kupfernitratlösung. Man erwärmte die Suspension auf 90 °C. Anschließend gab man unter Beibehaltung der Temperatur innerhalb 4 Stunden 3000 ml einer konzentrierten, wässsrigen Natriumcarbonatlösung zu, bis ein konstanter pH-Wert von etwa 9,6 erreicht war. Man kühlte auf Raumtemperatur und filtrierte die hierbei erhaltene, grauschwarze Suspension ab und wusch mit 80 l Wasser nach. Anschließend trocknete man das Pulver 16 Stunden bei 120 °C. Danach wurde 2 Stunden bei 300 °C kalziniert.

Die Elementaranalyse des so hergestellten Katalysatorpulvers (Atomabsorptionsspektroskopie) ergab einen Kupfergehalt von 15,1 Gew.-% (berechnet als elementares Kupfer) und 1,0 Gew.-% Natrium (ebenfalls berechnet auf elementares Natrium).

Eine Untersuchung des Katalysatorpulvers mittels Transmissionselektronenmikroskopie zeigte, daß die abgeschiedenen Kupferpartikel im Wesentlichen Durchmesser unterhalb 10 nm aufwiesen.

100 g des in Beispiel 1 erhaltenen Katalysatorpulvers wurden mit 3 g Graphit und 1 g Magnesiumstearat zu Tabletten (Durchmesser = 20 mm, 2 mm Stärke) vorkompaktiert. Die Tabletten wurden dann durch ein Sieb mit einer Maschenweite mit 1 mm gedrückt und zu Tabletten (5 mm Durchmesser und 3 mm Stärke) gepresst. Der Seitendruck der Tabletten betrug 36 ± 4 N. Die Bestimmung des Seitendrucks erfolgte mit einem Gerät der Fa. Frank; Typen-Nr. 81557.

### Vergleichsbeispiel 1

133,93 g Kupfer(II)nitrat-trihydrat wurden zusammen mit 200 g Siliciumdioxid (BET-Oberfläche 372 m²/g) in 1500 ml destilliertem Wasser suspendiert. Die Suspension wurde auf 80 °C erwärmt. Unter Beibehaltung der Temperatur gab man über einen Zeitraum von 2 Stunden tropfenweise 500 ml einer 0,3 N wässrigen Kaliumhydroxid-Lösung zu. Anschließend wurden innerhalb 4 Stunden 4200 ml einer 0,3 N wässrigen Kaliumhydroxid-Lösung zugegeben, bis ein konstanter pH-Wert von etwa 9,5 erreicht wurde. Die Temperatur von 90 °C unter Rühren vier Stunden beibehalten. Anschließend filtrierte man den grau-schwarzen Katalysator ab und trocknete 8 Stunden bei 120 °C. Danach wurde 5 Stunden bei 300 °C kalziniert.

Die Elementaranalyse des so hergestellten Katalysatorpulvers (Atomabsorptionsspektroskopie) ergab einen Kupfergehalt von 14,3 Gew.-% (berechnet als elementares Kupfer) und 3,3 Gew.-% Kalium (ebenfalls berechnet auf elementares Kalium).

Eine Untersuchung des Katalysatorpulvers mittels Transmissionselektronenmikroskopie zeigte, daß die abgeschiedenen Kupferpartikel im Wesentlichen Durchmesser bis zu 150 nm aufwiesen.

Das so erhaltene Katalysatorpulver wurde zusammen mit 1,5 g Graphit in der oben beschriebenen Weise zu Tabletten mit der Stärke 5x4 mm verpresst. Die Seitendruckfestigkeit der Tabletten lag bei 60 ± 8 N.

### II. Katalysatortest

Die Katalysatortests wurden in einem Rohrreaktor mit 5 cm Durchmesser und 60 cm Länge durchgeführt. Es wurden jeweils 200 ml des Katalysators aus I eingebaut und vor der Reaktion mit Wasserstoff aktiviert. Der Katalysator wurde vor der Beaufschlagung mit dem Edukt bei 120 °C mit 150 l N₂/h und 1,5 l H₂/h aktiviert. Wenn die Temperatur um mehr als 10 °C anstieg, wurde der Wasserstoffstrom gestoppt. Die Temperatur wurde dann schrittweise um 20 °C bis auf 200 °C angehoben, die Wasserstoffmenge wurde konstant gehalten. Bei 200 °C wurde der Katalysator dann mit 150 l N₂/h und 7,5 l H₂/h aktiviert. Nach der Aktivierung wurde der Katalysator mit dem Anol/Anolon-Gemisch (96 % Cyclohexanol, 4 % Cyclohexanon) beaufschlagt, wobei die LHSV etwa 0,7 h-¹ betrug. Die Reaktorausträge wurden nach verschiedenen Zeiten gaschromatographisch analysiert. Die Ergebnisse sind in Tabelle 1 zusammengefasst.

**Tabelle 1**

| Katalysator | Temperatur [°C] | Zeit [h] | Umsatz [%] | Selektivität [%] |
|---|---|---|---|---|
| Beispiel 1 | 217 | 34 | 49 | 99,4 |
| | 221 | 106 | 47 | 99,6 |
| | 234 | 322 | 48 | 99,4 |
| | 235 | 466 | 50 | 99,9 |
| | 235 | 562 | 51 | 99,9 |
| Vergleichsbeispiel 1 | 224 | 34 | 7 | 99,4 |
| | 236 | 58 | 12 | 99,5 |
| | 237 | 106 | 10 | 99,4 |
| | 237 | 226 | 10 | 99,5 |
| | 236 | 346 | 9 | 99,3 |

Der erfindungsgemäße Katalysator zeigt bereits bei > 220 °C einen Umsatz nahe des Gleichgewichtes bei gleichzeitig sehr hoher Selektivität > 99 %. Bei dieser Temperatur findet nur eine sehr langsame Desaktivierung des Katalysators, die sich in einer geringen Temperaturerhöhung zur Erhaltung des Umsatzes ausdrückt, statt.

## Patentansprüche

1. Verfahren zur Herstellung von oxidischen Katalysatoren, die Kupfer in einer Oxidationsstufe > 0 enthalten, durch Behandeln eines festen, oxidischen Trägermaterials mit einer wässrigen Lösung, die wenigstens ein Kupfersalz enthält und anschließendes Kalzinieren, **dadurch gekennzeichnet, dass** das Kupfer durch Fällung aus der wässrigen Lösung mittels eines Fällungsmittels auf das feste, oxidische Trägermaterial aufgebracht wird, wobei die wässrige Lösung wenigstens ein organisches, wasserlösliches Polymer in einer Konzentration von 0,1 bis 100 g/l enthält, welches Kupferionen koordinativ bindet und das ausgewählt ist unter Homo- und Copolymeren des N-Vinylpyrrolidons.

2. Verfahren nach Anspruch 1, **dadurch gekennzeichnet, dass** man die Fällung bei einer Temperatur oberhalb 50°C durchführt.

3. Verfahren nach einem der vorhergehenden Ansprüche, **dadurch gekennzeichnet, dass** das oxidische Trägermaterial eine BET-Oberfläche > 50 m²/g (gemessen nach DIN 66131) aufweist.

4. Verfahren nach einem der vorhergehenden Ansprüche, **dadurch gekennzeichnet, dass** das oxidische Trägermaterial wenigstens 70 Gew.-% SiO₂, bezogen auf das Gesamtgewicht des Trägermaterials enthält.

5. Verfahren nach einem der vorhergehenden Ansprüche, **dadurch gekennzeichnet, dass** man das Kupfer in einer Menge von 0,1 bis 50 Gew.-%, bezogen auf das Gesamtgewicht des Katalysators, auf das Trägermaterial aufbringt.

6. Katalysator enthaltend Kupfer in einer Oxidationsstufe > 0 auf einem festen oxidischen Trägermaterial, erhältlich nach einem Verfahren gemäß einem der vorhergehenden Ansprüche, wobei das auf dem Träger abgeschiedene Kupfer eine mittlere Partikelgröße unterhalb 20 nm aufweist.

7. Verfahren für die Dehydrierung von sekundären Alkoholen zu den entsprechenden Ketonen, **dadurch gekennzeichnet, dass** man die Dehydrierung an einem Katalysator gemäß Anspruch 6 durchführt.

8. Verfahren gemäß Anspruch 7, **dadurch gekennzeichnet, dass** der sekundäre Alkohol Cyclohexanol ist.

## Claims

1. A process for preparing an oxidic catalyst comprising copper in an oxidation state > 0, which comprises treating a solid oxidic support material with an aqueous solution comprising at least one copper salt and then calcining, wherein the copper is applied to the solid oxidic support material by precipitation from the aqueous solution using a precipitant, the aqueous solution comprising in a concentration of from 0.1 to 100 g/l at least one organic water soluble polymer which binds copper ions coordinatively and is selected from N-vinylpyrrolidone homopolymers and copolymers.

2. A process as claimed in claim 1, wherein the precipitation is carried out at a temperature above 50°C.

3. A process as claimed in either of the preceding claims, wherein the oxidic support material has a BET surface area > 50 m²/g (as measured according to DIN 66131).

4. A process as claimed in any of the preceding claims, wherein the oxidic support material comprises at least 70% by weight of SiO₂, based on the total weight of the support material.

5. A process as claimed in any of the preceding claims, wherein the copper is applied to the support material in an amount of from 0.1 to 50% by weight, based on the total weight of the catalyst.

6. A catalyst comprising copper in an oxidation state > 0 on a solid oxidic support material, obtainable by a process as claimed in any of the preceding claims, the copper precipitated on the support having an average particle size of below 20 nm.

7. A process for dehydrogenating a secondary alcohol to the corresponding ketone, which comprises effecting the dehydrogenation over a catalyst as claimed in claim 6.

8. A process as claimed in claim 7, wherein the secondary alcohol is cyclohexanol.

## Revendications

1. Procédé de préparation de catalyseurs oxydiques, contenant du cuivre à un étage d'oxydation supérieur à 0, par traitement d'un matériau de support oxydique solide par une solution aqueuse contenant au moins un sel de cuivre, et calcination subséquente, **caractérisé en ce que** le cuivre est appliqué sur le matériau de support oxydique solide par précipitation à partir de la solution aqueuse au moyen d'un réactif de précipitation, la solution aqueuse contenant au moins un polymère organique soluble dans l'eau, à une concentration de 0,1 à 100 g/l, lequel fixe les ions cuivre et est choisi parmi des homopolymères et des copolymères de la N-vinylpyrrolidone.

2. Procédé selon la revendication 1, **caractérisé en ce que** l'on opère la précipitation à une température supérieure à 50°C.

3. Procédé selon l'une des revendications qui précèdent, **caractérisé en ce que** le matériau de support oxydique présente une surface BET supérieure à 50 m²/g (mesurée selon DIN 66131).

4. Procédé selon l'une des revendications qui précèdent, **caractérisé en ce que** le matériau de support oxydique contient au moins 70% en poids de SiO₂, par rapport au poids total du matériau de support.

5. Procédé selon l'une des revendications qui précèdent, **caractérisé en ce que** le cuivre est appliqué en quantité de 0,1 à 50% en poids, par rapport au poids total du catalyseur, sur le matériau de support.

6. Catalyseur contenant du cuivre à un étage d'oxydation supérieur à 0 sur un matériau de support oxydique solide, que l'on peut obtenir par un procédé selon l'une des revendications qui précèdent, où le cuivre déposé sur le support présente une dimension particulaire moyenne de moins de 20 nm.

7. Procédé de déshydrogénation d'alcools secondaires en les cétones correspondantes, **caractérisé en ce que** l'on entreprend la déshydrogénation sur un catalyseur selon la revendication 6.

8. Procédé selon la revendication 7, **caractérisé en ce que** l'alcool secondaire est du cyclohexanol.
